# FASCICULE DE BREVET EUROPEEN

(11) **EP 2 475 637 B1**
(45) Date de publication et mention de la délivrance du brevet: **24.07.2019**
(21) Numéro de dépôt: 10770549.3
(22) Date de dépôt: 10.09.2010
(51) Int. Cl.: C07C 67/03, C07C 69/732, C11C 3/00, C11C 3/04, C08G 18/36

(54) **NOUVEAUX DÉRIVÉS D'HUILE DE RICIN ET LEUR PROCÉDÉ DE PRÉPARATION**
NEUE CASTORÖLDERIVATE UND HERSTELLUNGSVERFAHREN DAFÜR
NOVEL CASTOR OIL DERIVATIVES AND METHOD FOR THE PRODUCTION THEREOF

(30) Priorité: 11.09.2009 FR 0956258
(43) Date de publication de la demande: 18.07.2012
(73) Titulaire: Centre National de la Recherche Scientifique (C.N.R.S.), 75016 Paris (FR)
(72) Inventeur: CRAMAIL, Henri, F-33350 Sainte Terre (FR); BOYER, Aurélie, F-33000 Bordeaux (FR); CLOUTET, Eric, F-33880 Saint Caprais De Bordeaux (FR); ALFOS, Carine, F-33600 Pessac (FR)
(74) Mandataire: Lavoix
(86) Numéro de dépôt international: PCT/FR2010/051893
(87) Numéro de publication internationale: WO 2011/030075

(56) Documents cités:
- US-A- 2 902 500
- US-A- 4 125 545
- US-A- 5 306 788
- LYON, CAMERON K. ET AL: "Diol diricinoleates from dihaloalkanes", JOURNAL OF THE AMERICAN OIL CHEMISTS' SOCIETY, vol. 47, no. 4, 1970, pages 145-146, XP002565322,
- PENA, R. ET AL.: "Transesterification of Castor Oil: Effect of Catalyst and Co-Solvent", INDUSTRIAL & ENGINEERING CHEMISTRY RESEARCH, vol. 48, no. 3, 30 septembre 2008 (2008-09-30), pages 1186-1189, XP002616422, ISSN: 1520-5045, DOI: 10.1021/ie8005929

## Description

La présente invention a pour objet de nouveaux dérivés d'huile de ricin ainsi que leur procédé de préparation.

Il existe différentes approches pour la synthèse de polymères à partir d'huiles végétales. La première, la plus répandue, consiste à considérer les triglycérides comme matériaux de base, ceux-ci pouvant être époxydés puis par exemple alcoolisés ou hydroformylés, afin de les rendre fonctionnels et polymérisables.

Une huile est un mélange de triglycérides (triesters) formés par condensation des acides gras et du glycérol. Le nombre élevé de types d'acides gras (jusqu'à 24) présents dans chaque corps gras et les multiples possibilités de leurs combinaisons avec les molécules de glycérol font que les corps gras sont des mélanges très complexes de composés dont les propriétés varient d'une huile à une autre. La nature des triglycérides peut donc varier au sein d'une même huile.

Les sites réactifs présents dans un triglycéride sont principalement les doubles liaisons et les fonctions ester. La réactivité des doubles liaisons permet d'introduire des fonctions hydroxyles, permettant ainsi l'accès à des monomères plurihydroxylés. Il est néanmoins impossible d'obtenir des triglycérides ayant des structures et des fonctionnalités parfaitement définies.

US 5 306 788 décrit la préparation de polyesters par réaction d'esters avec des diols. Dans ce document, les composés obtenus comprennent des fonctions esters reliées entre elles par des radicaux alkylènes.

La présente invention a pour but de fournir un procédé de préparation de composés à partir d'huile végétale permettant de s'affranchir des inconvénients susmentionnés.

La présente invention a également pour but de fournir un procédé de préparation de composés dérivés d'huile végétale comprenant l'utilisation de catalyseurs répondant mieux aux attentes environnementales que la plupart des catalyseurs homogènes utilisés et qui limitent les réactions secondaires.

La présente invention a également pour but de fournir un procédé qui, contrairement aux procédés de l'art antérieur qui concernent la transformation chimique à partir de triglycérides ayant des structures mal définies, consiste en une voie simple et efficace de modification chimique de monoesters ou de triglycérides pour l'obtention de précurseurs fonctionnels à fonctionnalité contrôlée.

Un but de la présente invention est de fournir un procédé simple permettant l'accès à de nouveaux synthons, mono-esters ou di-esters, possédant des structures bien définies et faisant appel à une catalyse propre.

La présente demande décrit notamment des composés de formule (I) suivante : 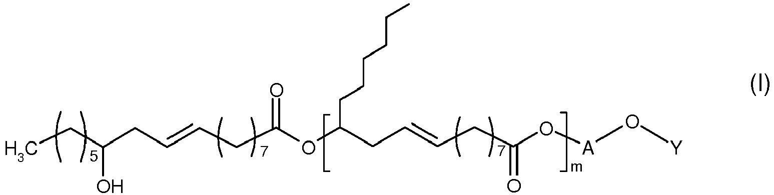 dans laquelle :
- m est égal à 0 ou est un nombre entier n compris de 1 à 20 ;
- A représente un radical alkylène divalent linéaire ou ramifié, comprenant de 1 à 10 atomes de carbone, et éventuellement interrompu par un ou plusieurs hétéroatomes, et plus particulièrement par un ou plusieurs atomes d'oxygène, et
- Y représente un atome d'hydrogène ou un groupe de formule (II) 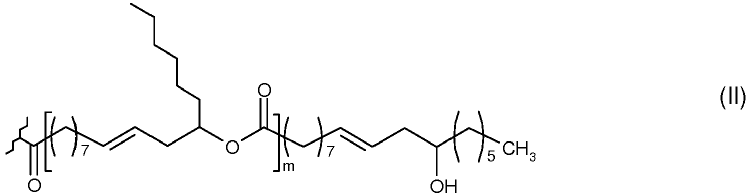 m étant tel que défini ci-dessus dans la formule (I).

Ainsi, dans la formule (I), lorsque m est égal à 0, la présente demande concerne des composés de formule (Ibis) suivante : 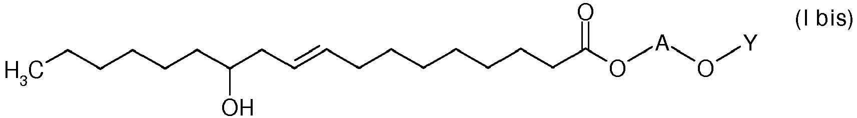 A et Y étant tels que définis ci-dessus dans la formule (I).

Parmi les composés décrits dans la demande, on peut notamment citer les composés monoesters de formule (I-1) suivante : 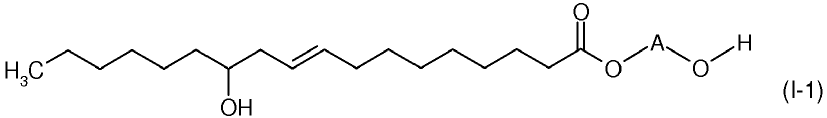 A étant tel que défini ci-dessus dans la formule (I).

De préférence, dans la formule (I-1), A représente un radical alkylène divalent linéaire ou ramifié, comprenant de 1 à 10 atomes de carbone, interrompu par au moins un atome d'oxygène.

On peut également citer les composés diesters de formule (I-2) suivante : 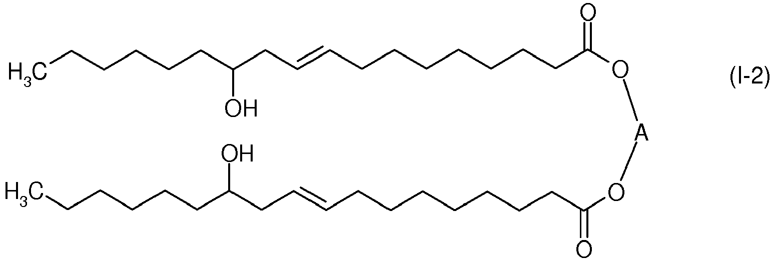 A étant tel que défini ci-dessus dans la formule (I).

De préférence, dans la formule (I-2), A représente un radical alkylène divalent linéaire ou ramifié, comprenant de 1 à 10 atomes de carbone, interrompu par au moins un atome d'oxygène.

La présente demande décrit également les composés de formule (I-3) suivante : 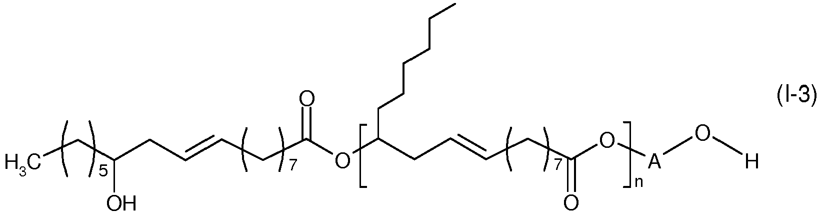 n et A étant tels que définis ci-dessus dans la formule (I).

La présente invention concerne les composés de formule (I-4) suivante : 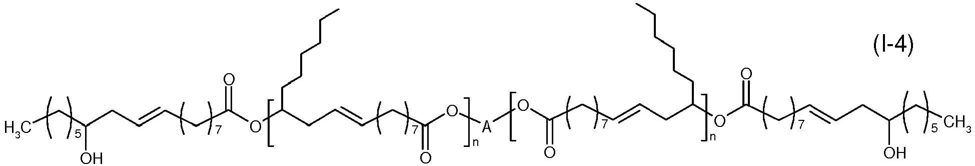 n et A étant tels que définis ci-dessus dans la formule (I).

Selon un mode de réalisation préféré, la présente invention concerne également des composés répondant à la formule (I-4) dans laquelle A représente un radical alkylène comprenant 3 ou 4 atomes de carbone.

Selon un mode de réalisation préféré, la présente invention concerne également des composés répondant à la formule (I-4) dans laquelle A représente un radical (poly)propylèneglycol.

La présente demande décrit également un procédé de préparation d'un composé de formule (I-1) telle que définie ci-dessus, comprenant une étape de transestérification d'huile de ricin avec un diol de formule (III) suivante HO-A-OH, A étant tel que défini ci-dessus dans la formule (I-1).

La présente demande décrit également un procédé de préparation d'un composé de formule (I-1) telle que définie ci-dessus, comprenant une étape de transestérification d'huile de ricin avec un diol de formule (III) suivante HO-A-OH, A représentant un radical alkylène divalent linéaire ou ramifié, comprenant de 1 à 10 atomes de carbone, interrompu par au moins un atome d'oxygène.

Selon un mode de réalisation avantageux, l'étape de transestérification est effectuée en présence d'un catalyseur de transestérification.

Selon un mode de réalisation particulièrement avantageux, le catalyseur de transestérification mis en oeuvre dans le cadre du procédé est choisi dans le groupe constitué de l'oxyde de magnésium, de l'acétate de zinc, du tétraalkoxyde de titane, notamment du tétrabutoxyde de titane, et du méthanolate de sodium.

De préférence, pour préparer les composés de formule (I-1) susmentionnée, on utilise le diol de formule (III) en excès. Ainsi, plus particulièrement, le ratio entre le nombre de moles du diol et le nombre de moles d'huile de ricin est compris de 10 à 100.

La présente demande décrit également un procédé de préparation d'un composé de formule (I-2) telle que définie ci-dessus, comprenant les étapes suivantes :
- une étape a) de transestérification d'huile de ricin avec un alcool ROH, R représentant un groupe alkyle linéaire ou ramifié, comprenant de 1 à 10, de préférence de 1 à 6, atomes de carbone, pour obtenir un composé de formule (IV) suivante : 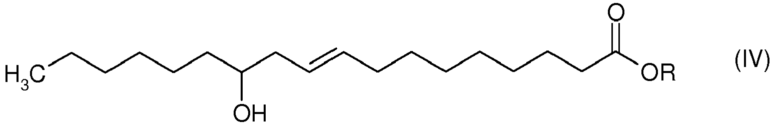
- et une étape b) de transestérification du composé de formule (IV) avec un diol de formule (III) suivante HO-A-OH, A représentant un radical alkylène divalent linéaire ou ramifié, comprenant de 1 à 10 atomes de carbone, et éventuellement interrompu par un ou plusieurs hétéroatomes, et plus particulièrement par un ou plusieurs atomes d'oxygène.

Selon un mode de réalisation préféré, l'étape b) du procédé est effectuée en présence d'un catalyseur de transestérification.

De façon particulièrement avantageuse, ledit catalyseur de transestérification est choisi dans le groupe constitué de l'oxyde de magnésium, de l'acétate de zinc, du tétraalkoxyde de titane, de préférence du tétrabutoxyde de titane, et du méthanolate de sodium.

De préférence, pour préparer les composés de formule (I-2) susmentionnée, on utilise le diol de formule (III) en défaut. Ainsi, plus particulièrement, le ratio entre le nombre de moles du diol et le nombre de moles d'huile de ricin est compris de 0,2 à 3.

La présente demande décrit également un procédé de préparation d'un composé de formule (I-2) 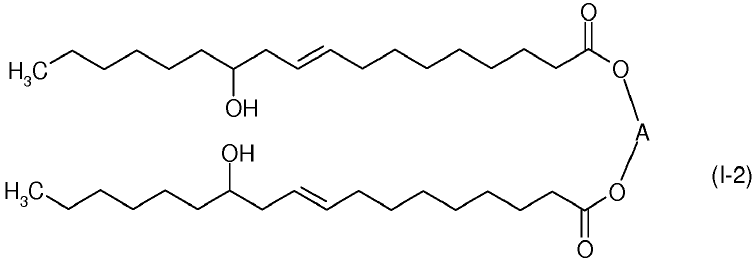 A étant tel que défini ci-dessus dans la formule (I),
ledit procédé comprenant les étapes suivantes :
- une étape a) de transestérification d'huile de ricin avec un alcool ROH, R représentant un groupe alkyle linéaire ou ramifié, comprenant de 1 à 10, de préférence de 1 à 6, atomes de carbone, pour obtenir un composé de formule (IV) suivante : 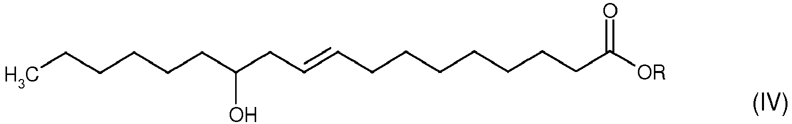
- une étape a1) de réaction du composé (IV) avec du méthanolate de sodium pour obtenir un composé de formule (V) suivante : 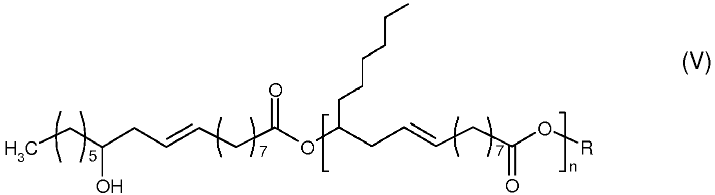 n étant tel que défini ci-dessus dans la formule (I),
- et une étape b) de transestérification du composé de formule (V) avec un diol de formule (III) suivante HO-A-OH, A représentant un radical alkylène divalent linéaire ou ramifié, comprenant de 1 à 10 atomes de carbone, et éventuellement interrompu par un ou plusieurs hétéroatomes, et plus particulièrement par un ou plusieurs atomes d'oxygène.

De préférence, selon le procédé de préparation de la présente demande, l'étape a1) est effectuée à une température comprise de 100°C à 220°C.

Dans le cadre des procédés selon la présente demande, il est particulièrement avantageux d'effectuer les réactions en catalyse hétérogène, par exemple avec l'oxyde de magnésium, ce qui permet de filtrer le catalyseur après la réaction, et donc de l'éliminer du milieu réactionnel.

Selon un autre mode de réalisation avantageux, les composés décrits dans la présente demande sont préparés selon un procédé mettant en oeuvre un catalyseur à base de titane, et plus particulièrement le Ti(OBu)₄ qui présente des propriétés intéressantes en termes de viscosité.

Les composés susmentionnés de l'invention sont des polyols contenant au moins deux fonctions OH peuvent être utilisés, entre autres, comme monomères. Leur pureté permet d'optimiser les propriétés des polymères obtenus.

Ainsi, des polyuréthanes ont ensuite été synthétisés par polymérisation en masse de ces polyols avec l'IPDI (ou par exemple également avec du MDI ou de l'HDI), à 60°C en présence de dibutyl dilaurate d'étain. La formation des polyuréthanes est confirmée par FTIR avec la disparition de la bande de vibration de l'isocyanate. La chromatographie d'exclusion stérique confirme des masses molaires comprises entre 14 000 et 50 000 g/mol. Ces monomères di-OH peuvent également être utilisés pour la synthèse d'autres polymères tels que des polyesters, polyéthers, polycarbonates, etc.

Les composés de formule (I), (I-1) ou (I-2) sont notamment utilisés pour réagir avec des polyisocyanates pour la préparation de polymères, notamment polyuréthanes.

Ainsi, ces composés peuvent être utilisés pour la préparation de mousses rigides, d'isolants électriques, de revêtements, d'adhésifs, de mousses flexibles (notamment dans le domaine de l'ameublement ou de l'automobile) ou de semelles de chaussures.

Plus exactement, les composés selon la présente demande sont utilisés pour la préparation de mousses rigides en les faisant réagir avec des polyisocyanates en présence d'un catalyseur et d'un agent moussant (auxquels on peut également ajouter des surfactants, des colorants, des antioxydants, des conservateurs, des agents plastifiants, des agents de réticulation, des ignifuges, etc.).

De préférence, on peut préparer une telle mousse rigide en faisant réagir ensemble les constituants suivants : 60 g de polyisocyanate, 40 g de polyol, 1,2 g d'eau (agent moussant), 0,1-0,4 g de catalyseur et 1-4 g de surfactant.

Plus exactement, les polyols selon la présente demande sont utilisés pour la préparation d'isolants électriques en les faisant réagir avec des polyisocyanates en présence d'un agent anti-mousse et d'un agent séchant.

De préférence, un tel isolant électrique peut être préparé en faisant réagir ensemble 60 g de polyol, 29 g de polyisocyanate, 0,6 g d'agent anti-mousse et 3 g d'agent séchant, et éventuellement 60 g de charges (silice).

Plus exactement, les polyols selon la présente demande sont utilisés pour la préparation de revêtements en les faisant réagir avec des polyisocyanates. Par exemple, des revêtements sont préparés en utilisant les polyols et les polyisocyanate purs, ou en utilisant les polyols et les polyisocyanate avec solvants (on peut également ajouter des colorants, des pigments, des charges, des additifs rhéologiques, des antioxydants, des bactéricides, des fongicides, des inhibiteurs de corrosion, des catalyseurs ou des stabilisateurs UV).

Pour la préparation d'adhésifs selon la présente demande, on prévoit également d'utiliser les polyols de l'invention purs avec des polyisocyanates purs.

En ce qui concerne les mousses flexibles, de préférence, on utilise 60 g de polyol selon l'invention, 100 g d'isocyanate, 4.5 g d'eau (agent moussant), 0,12 g de catalyseur 1, 0,38 g de catalyseur 2 et 3 g de surfactant.

Enfin, une formulation spécifique selon la demande pour la préparation de semelles de chaussures comprend 59 g d'isocyanate, 94,5 g de polyol selon l'invention, 4,1 g d'éthylène glycol et 1,4 g de catalyseur.

La présente invention concerne également les polymères tels qu'obtenus par polymérisation d'un composé selon la présente invention et d'un (poly)isocyanate.

### PARTIE EXPÉRIMENTALE

### Exemple 1 : Préparation d'esters de butanediol

Cet exemple concerne la préparation du composé (1) de formule suivante : 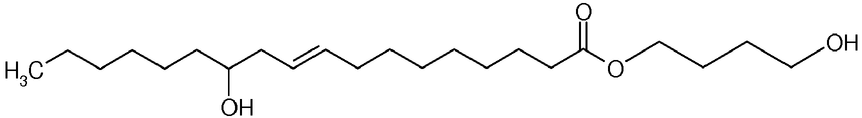 Il s'agit d'un composé de formule (I-1) dans laquelle A représente un radical butylène.

Le produit de départ est l'huile de ricin de formule :

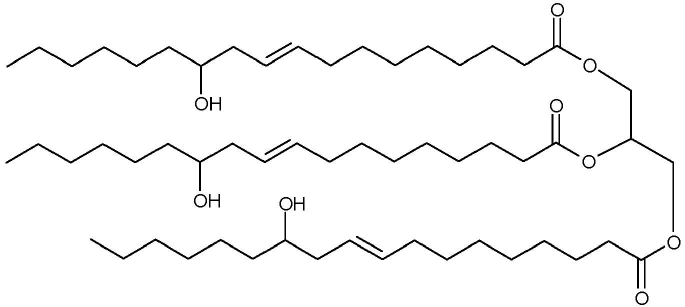

L'huile de ricin utilisé présente la composition massique suivante en acides gras :

| | |
|---|---|
| C16:0 | 1,1% |
| C18:0 | 1,3% |
| C18:1 | 3,4% |
| C18:2 | 4,5% |
| C18:3 | 0,1% |
| C18:OH | 88,5% |
| C20:0 | 0,1% |
| C20:1 | 0,5% |
| C22:0 | 0,5% |

On a introduit dans un réacteur (2 litres) 167,8 g d'huile de ricin (ITERG, M = 928 g.mol⁻¹ - indice d'acide = 0,65% - point éclair = 229°C) avec 1 556,4 g de 1,4-butanediol (Aldrich - M = 90,12 g.mol⁻¹) et 2,4 g de catalyseur MgO (Fluka). Le butanediol est donc en excès par rapport à l'huile de ricin avec un ratio molaire butanediol/huile de ricin d'environ 100.

L'ensemble est mélangé sous agitation à 500 tr.min⁻¹ et chauffé jusqu'à 150°C. On a alors observé une coloration jaune pâle.

Ensuite, l'eau résiduelle a été condensée vers 100°C et l'ensemble a été chauffé à 150-155°C pendant 10 heures. Enfin, on a laissé une nuit au repos le mélange réactionnel dans le réacteur.

Pour éliminer le butanediol, on a effectué une distillation sous vide sous diazote et le milieu réactionnel a été centrifugé à 500 tr.min⁻¹ pendant 3 cycles de 15 minutes. On a également filtré le catalyseur (MgO) sous vide sur Büchner.

Afin de récupérer le produit final, on a effectué une distillation sous pression réduite dudit produit à une température de 110-114°C sous une pression de 3 à 5 mbar.

On a ainsi obtenu 150 g d'une huile jaune translucide. Ce produit présente un indice d'acide de 1,07% et un indice d'hydroxyle de 273,1 mg KOH/g.

D'après la caractérisation par chromatographie liquide HPLC effectuée, le produit final obtenu présente la composition suivante :

| | |
|---|---|
| Huile de ricin | 1,5% |
| Diester - composé de formule (I-2) | 4,7% |
| **Monoester** - **composé (1)** | **88,5%** |
| Alcool résiduel | 4,2% |
| Acide gras libre | 0,7% |
| Polymères | 0,0% |

### Exemple 2 : Préparation d'esters de propanediol

Cet exemple concerne la préparation du composé (**2**) de formule suivante : 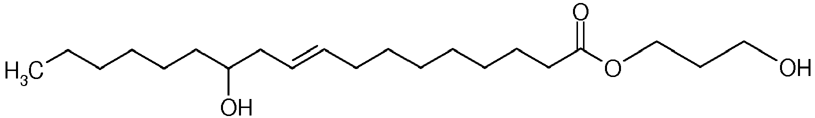 Il s'agit d'un composé de formule (I-1) dans laquelle A représente un radical propylène.

Le produit de départ est l'huile de ricin telle que décrite dans l'exemple 1.

On a introduit dans un réacteur (4 litres) 250,5 g d'huile de ricin (ITERG, M = 928 g.mol⁻¹ - indice d'acide = 0,65% - point éclair = 229°C) avec 2 052,8 g de 1,3-propanediol (Aldrich - M = 76,09 g.mol⁻¹) et 3,9 g de catalyseur MgO (Fluka). Le propanediol est donc en excès par rapport à l'huile de ricin avec un ratio molaire propanediol/huile de ricin d'environ 100.

L'ensemble est mélangé sous agitation à 650 tr.min⁻¹ et chauffé jusqu'à 150°C. On a alors observé une coloration jaune pâle.

Ensuite, l'eau résiduelle a été condensée vers 100°C et l'ensemble a été chauffé à 150°C pendant 8 heures.

On a ensuite laissé décanter l'ensemble dans une ampoule pour obtenir une phase homogène. Puis, on a effectué une extraction liquide-liquide avec de l'hexane et on a observé deux phases : une phase jaune supérieure (hexane) et une phase inférieure trouble (phase aqueuse). La phase aqueuse a alors été récupérée et lavée avec de l'hexane. On a également filtré les traces de catalyseur sur Büchner. Enfin, les solvants ont été évaporés au rotavapor puis lavés à l'eau chaude (à 60°C) et de nouveau évaporés et séchés au rotavapor.

On a ainsi obtenu 213 g d'esters de propanediol. Ce produit présente un indice d'acide de 2,68 mg KOH/mg et un indice d'hydroxyle de 298,7 mg KOH/g.

D'après la caractérisation par chromatographie liquide HPLC effectuée, le produit final obtenu présente la composition suivante :

| | |
|---|---|
| Triglycérides | 1,3% |
| Diester - composé de formule (I-2) | 2,8% |
| **Monoester - composé (2)** | **96,0%** |
| Propanediol | 0,0% |
| Polymères | 0,0% |

On a également effectué une analyse CPG qui a permis de constater la composition massique suivante :

| | |
|---|---|
| Triglycérides | 0,3% |
| Diester - composé de formule (I-2) | 1,7% |
| **Monoester - composé (2)** | **98,0%** |
| Propanediol | 0,0% |
| Eau et volatils | 0,27% |

### Exemple 3 : Préparation de diesters de butanediol

Cet exemple concerne la préparation du composé (**3**) de formule suivante : 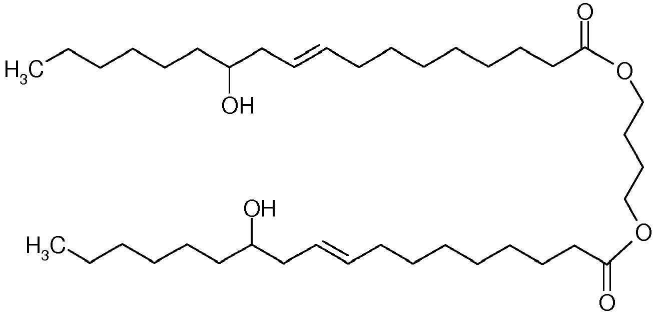 Il s'agit d'un composé de formule (I-2) dans laquelle A représente un radical butylène.

Le produit de départ est l'huile de ricin comme décrit dans l'exemple 1.

Le composé (3) a été obtenu selon un procédé en deux étapes.

### Première étape : Synthèse d'ester éthylique d'huile de ricin (4)

Cette étape consiste à préparer un ester éthylique d'huile de ricin de formule suivante :

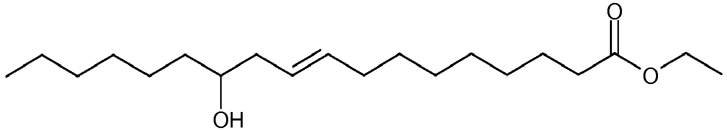

On a introduit dans un réacteur double enveloppe (de 1 litre) 402,4 g d'huile de ricin (ITERG, M = 928 g.mol⁻¹ - teneur en eau = 0,35% en poids) avec 405,4 g d'éthanol absolu (JT Baker - M = 46,07 g.mol⁻¹). L'ensemble est mélangé sous agitation à 650 tr.min⁻¹ et chauffé à 65°C. On a ensuite ajouté 4,5214 g de MeONa (Aldrich - M = 54,02 g.mol⁻¹) dans le réacteur et on a alors constaté un changement de couleur du produit et l'apparition d'un trouble instantané. L'ensemble est ensuite laissé réagir pendant 30 minutes à 50°C.

Le mélange réactionnel résultant a ensuite été transvasé dans une ampoule à décanter afin d'éliminer le glycérol et évaporer l'éthanol. On a ensuite effectué une neutralisation avec quelques gouttes de HCl puis un lavage à l'eau jusqu'à neutralité. Enfin, l'eau résiduelle a été distillée au rotavapor.

On a ainsi obtenu 360,2 g d'ester éthylique d'huile de ricin (4) avec une teneur en eau de 0,23% en poids.

D'après la caractérisation par chromatographie en phase gazeuse effectuée, on a obtenu une composition comprenant 93,8% en poids d'ester éthylique (4).

### Deuxième étape : Synthèse d'esters de butanediol d'huile de ricin

Le produit de départ est le composé (4) tel qu'obtenu à l'issue de la première étape.

On a introduit dans un réacteur (500 mL) 300,5 g de composé (4) avec 43,5 g (0,5 mol) de 1,4-butanediol (Aldrich)(composé de formule (III) avec A₂ = butylène). L'ensemble est chauffé à 65°C. On a ensuite ajouté 3,6005 g de MeONa (Aldrich) dans le réacteur et on a alors constaté un changement de couleur du produit (jaune opaque). L'ensemble est ensuite laissé réagir pendant 6 heures à 70-75°C sous agitation (650 tr.min⁻¹) à une pression de 800 à 2 mbar.

On a ensuite effectué une neutralisation avec quelques gouttes de HCl puis un lavage à l'eau pour éliminer les traces de butanediol jusqu'à neutralité. Enfin, l'eau résiduelle a été distillée au rotavapor.

On a ainsi obtenu 260 g d'ester de butanediol d'huile de ricin de formule (3) susmentionnée avec une teneur en eau de 0,30% en poids. Le produit de formule (3) est sous la forme d'un liquide jaune et présente un indice d'acide de 5,05%.

D'après la caractérisation par chromatographie en phase gazeuse effectuée, on a obtenu une composition comprenant 43,9% en poids de diesters (composé (3)), 30,4% en poids de monoesters et 25,7% en poids de composé (4).

### Exemple 4 : Préparation d'esters de butanediol

Le composé obtenu présente la même formule que celui de l'exemple 1 mais présente une composition différente.

On a introduit dans un réacteur (2 litres) 505,3 g d'huile de ricin avec 698,9 g de 1,4-butanediol (Aldrich - M = 90,12 g.mol⁻¹) et 7,5 g de catalyseur MgO (Fluka). Le butanediol est donc en excès par rapport à l'huile de ricin.

L'ensemble est mélangé sous agitation à 500 tr.min⁻¹ et chauffé jusqu'à 150°C. On a alors observé une coloration jaune pâle.

Ensuite, l'eau résiduelle a été condensée vers 100°C et l'ensemble a été chauffé à 150-155°C pendant 10 heures. Enfin, on a laissé une nuit au repos le mélange réactionnel dans le réacteur.

Le mélange réactionnel a été centrifugé à 4 500 tr.min⁻¹ pendant trois fois 15 minutes.

On a également filtré le catalyseur (MgO) sous vide sur Büchner.

Afin de récupérer le produit final, on a effectué une distillation sous pression réduite dudit produit à une température de 107°C sous une pression de 1 mbar.

On a ainsi obtenu 483 g d'une huile jaune orangée. Ce produit présente un indice d'acide de 0,95% et un indice d'hydroxyle de 283,1 mg KOH/g.

D'après la caractérisation par chromatographie liquide HPLC effectuée, le produit final obtenu présente la composition suivante :

| | |
|---|---|
| Huile de ricin | 4,2% |
| Diester - composé de formule (I-2) | 12,3% |
| **Monoester -** composé de formule (I-1) | **79,4%** |
| Alcool résiduel | 3,7% |
| Acide gras libre | 0,4% |
| Polymères | 0,0% |

### Exemple 5 : Préparation de polyricinoléate de butanediol

Cet exemple consiste à préparer un estolide d'huile de ricin (polyricinoléate de butanediol) de formule (I-4) avec A = -(CH₂)₄- en plusieurs étapes à partir d'huile de ricin.

### Première étape : Synthèse d'ester méthylique d'huile de ricin (5)

Cette étape consiste à préparer un ester méthylique d'huile de ricin de formule suivante :

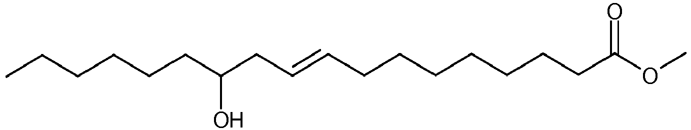

On a introduit dans un réacteur double enveloppe (de 30 litres) 16 980 g d'huile de ricin (ITERG, M = 928 g.mol⁻¹ - teneur en eau = 0,35% en poids) avec 4 393 g de méthanol (Baker) et 454,12 g de MeONa (Aldrich). L'ensemble est chauffé à 75°C. La température est maintenue à 75°C pendant 2 heures puis le milieu réactionnel est refroidi à 40°C.

Le mélange réactionnel résultant a ensuite été laissé décanter. On a ensuite effectué plusieurs lavages avec de l'eau et du HCl.

On a ainsi obtenu 15 990 g d'ester méthylique d'huile de ricin (5).

### Deuxième étape : Synthèse d'estolides d'huile de ricin

Le produit de départ est le composé (5) tel qu'obtenu à l'issue de la première étape.

On a introduit dans un réacteur (1 L) 890,0 g de composé (5) avec 13,9 g (1,56 % w/w) de MeONa (Sigma-Aldrich). L'ensemble est chauffé lentement sous vide de 100°C à 140°C, puis le milieu réactionnel est maintenu à 135°C pendant une heure à pression atmosphérique.

On a ainsi obtenu 841,5 g d'estolide de ricin sous la forme d'un liquide visqueux noir / marron très foncé.

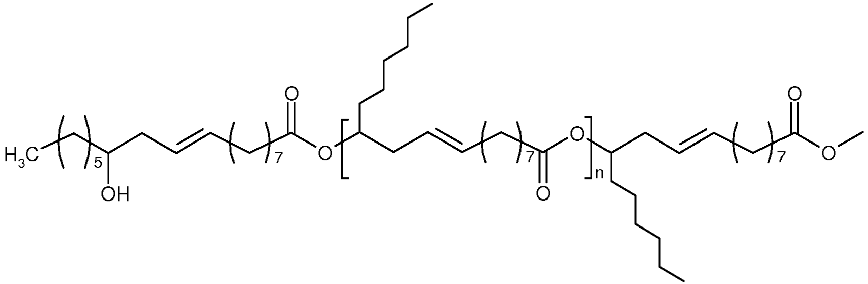

### Troisième étape : Synthèse d'estolides de ricin de butanediol

Cette étape consiste à préparer un estolide d'huile de ricin de formule (I-4) avec A = -(CH₂)₄-.

Le produit de départ est l'estolide de ricin obtenu à l'issue de l'étape précédente.

On a introduit dans un réacteur (1 L) 458,0 g d'estolide avec 13,5 g (0,1n) de butanediol (Sigma-Aldrich) et 2,2 g de MeONa (0,5% w/w). L'ensemble est chauffé lentement sous vide de 120°C à 140°C.

Plusieurs étapes de décantation / lavage ont été effectuées (avec HCl, NaCl et eau).

On a ainsi obtenu 320,5 g d'estolide de ricin de butanediol sous la forme d'un liquide orange / marron limpide.

### Exemple 6 : Préparation de polyricinoléate de butanediol

Cet exemple consiste à préparer un estolide d'huile de ricin de formule (I-4) avec A = -(CH₂)₄-.

Le produit de départ est le composé (5) tel qu'obtenu à l'issue de la première étape de l'exemple 5.

On a introduit dans un réacteur (250 mL) 150 g de composé (5) avec 4,35 g (0,1n) de 1,4-butanediol (Aldrich) et 1,5 g de MeONa (1% w/w). L'ensemble est chauffé à 140°C pendant 4 heures. Un vide (500-700 mbar) est appliqué pendant environ une heure et la pression est diminuée jusqu'à 150 mbar jusqu'à la fin de la réaction pour éliminer le méthanol généré. Une fois la réaction complète, le mélange réactionnel a été refroidi à température ambiante puis il a été dissous dans de l'acétate d'éthyle (200 mL). La solution a été lavée avec une solution d'HCl 37% et de l'eau jusqu'à neutralité. Le solvant a été éliminé sur un évaporateur rotatif pour donner un liquide jaune.

On a ainsi obtenu 110,5 g d'estolide de ricin de butanediol (polyricinoléate de butanediol).

D'après la caractérisation par chromatographie en phase gazeuse effectuée, on a obtenu une composition comprenant 56,40% en poids de polymère estolide.

Ce protocole a été appliqué pour la synthèse d'estolides de ricin à partir de butanediol en faisant varier les quantités de butanediol, comme indiqué dans le tableau ci-dessous :

| | Masse de composé (5) (g) | Masse de butanediol (g) | Masse de MeONa (g) | Masse de produit final (g) |
|---|---|---|---|---|
| Ex. 6.1 | 220 | 12,8 | 2,2 | 76,6 |
| Ex. 6.2 | 220 | 19,14 | 2,2 | 146,3 |
| Ex. 6.3 | 220 | 25,52 | 2,2 | 194,7 |
| Ex. 6.4 | 220 | 31,91 | 2,2 | 196,1 |

Le produit final est obtenu sous la forme d'un mélange comprenant des composés de type monomère, de type dimère et de type estolide (polyricinoléate de butanediol).

### Exemple 7 : Préparation de polyricinoléate de polypropylène glycol

Cet exemple consiste à préparer un composé de formule (I-4) où A représente un motif polypropylène glycol.

Le produit de départ est le composé (5) tel qu'obtenu à l'issue de la première étape de l'exemple 5.

On a introduit dans un réacteur (1 L) 500 g (1,61 mol) de composé (5) avec 64,37 g (0,1 éq) de Voranol P400 (Bostik) et 5,48 g de Ti(OBu)₄ (1% n/n)(Sigma-Aldrich). L'ensemble est chauffé sous vide sous 200°C pendant 4 heures.

La réaction est menée sous 10 mbar pour éliminer le méthanol généré. Une fois la réaction finie, le polyol est chauffé à température ambiante pour donner un liquide jaune.

On a ainsi obtenu 475,2 g d'estolide de ricin de voranol.

Ce protocole a été appliqué pour la synthèse d'estolides de ricin à partir de voranol en faisant varier les quantités de voranol, comme indiqué dans le tableau ci-dessous :

| | Masse de composé (5) (g) | Masse de voranol (g) | Masse de Ti(OBu)₄ (g) | Masse de produit final (g) |
|---|---|---|---|---|
| Ex. 7.1 | 500 | 128,74 | 5,48 | 535,7 |
| Ex. 7.2 | 400 | 154,49 | 4,38 | 472,9 |
| Ex. 7.3 | 350 | 180,24 | 3,83 | 453,3 |
| Ex. 7.4 | 320 | 205,99 | 3,51 | 452 |

### Exemple 8 : Préparation de polymères (polyuréthanes) à partir des polyols de l'invention

Les polyols de l'invention sont utilisés pour préparer des polymères par exemple par réaction avec des isocyanates. Le protocole appliqué est décrit ci-après et peut être appliqué à tout polyol et tout isocyanate.

On a ajouté dans un réacteur d'un litre le polyol de l'invention et le catalyseur puis l'isocyanate (en particulier l'IPDI ou le HDMI) a été additionné dans le réacteur par l'intermédiaire d'un entonnoir. L'ensemble a ensuite été agité à 80 t.min⁻¹ sous diazote afin d'homogénéiser le mélange. On a alors observé l'apparition de bulles dans le mélange réactionnel et on a maintenu la température du mélange à 60°C par chauffage.

On a effectué un suivi cinétique de la réaction par analyse IR ce qui a permis d'observer la disparition de la bande N=C à 2 269,94 cm⁻¹ et l'apparition de la bande N-H à 3 350 cm⁻¹.

Plus particulièrement, ce protocole a été appliqué en utilisant comme polyol un monoester de butanediol d'huile de ricin (composé monoester de l'exemple 4) et en faisant varier la nature de l'isocyanate (IPDI et HMDI), ainsi que le temps de réaction et le ratio OH:NCO.

Le catalyseur utilisé est le DBTDL (LCPO)(dilaurate de dibutylétain) à 0,1% en poids.

Les résultats obtenus sont résumés ci-après dans les tableaux 1 et 2.

**Le tableau 1 correspond à la synthèse de polyuréthane par réaction avec l'IPDI (Aldrich - M = 222,29 g.mol⁻¹)(isophorone diisocyanate) :**

| N° | OH:NCO | Durée de réaction (h) | Solubilité (DCM, THF, DMF) | Analyse IR | Analyse CPG (Mw) | Viscosité (cst) | | | |
|---|---|---|---|---|---|---|---|---|---|
| | | | | | | 80°C | | 100°C | |
| | | | | | | Cisaillement (s⁻¹) | | | |
| | | | | | | 1 | 10 | 1 | 10 |
| 1 | 1:0,3 | 6 | soluble | Pas de bande isocyanate | 1 290 | - | 18 | - | 12,3 |
| 2 | 1:0,5 | 6 | soluble | Pas de bande isocyanate | 1 890 | - | 41,5 | - | 19 |
| 3 | 1:0,66 | 4 | soluble | Pas de bande isocyanate | 2210 | 35 | 19,5 | - | 11,5 |
| 4 | 1:0,76 | 4 | soluble | Pas de bande isocyanate | 3710 | 275 | 205 | - | 75 |
| 5 | 1:0,87 | 4 | soluble | Pas de bande isocyanate | 5620 | 1 015 | 1 088 | 278 | 265 |
| 6 | 1:0,95 | 6 | soluble | Pas de bande isocyanate | 6640 | 2 927 | 2 839 | 531 | 563 |
| 7 | 1:1 | 6 | soluble | Pas de bande isocyanate | 9470 | - | - | - | - |
| 8 | 1:1,04 | 6 | soluble | Pas de bande isocyanate | 10 750 | - | - | - | - |
| 9 | 1:1,15 | 6 | insoluble | - | - | - | - | - | - |

| | | | | | | | | | |
|---|---|---|---|---|---|---|---|---|---|
| DCM : dichlorométhane THF : tétrahydrofurane DMF : diméthylformamide | | | | | | | | | |

Pour obtenir des polyuréthanes de viscosité satisfaisante, il est donc préférable de travailler à des ratios OH:NCO inférieurs à 1.

**Le tableau 2 correspond à la synthèse de polyuréthane par réaction avec le HMDI (hexaméthylène diisocyanate) :**

| N° | OH:NCO | Durée de réaction (h) | Solubilité (DCM, THF, DMF) | Analyse IR | Analyse CPG (Mw) | Viscosité (cst) | | | |
|---|---|---|---|---|---|---|---|---|---|
| | | | | | | 80°C | | 100°C | |
| | | | | | | Cisaillement (s⁻¹) | | | |
| | | | | | | 1 | 10 | 1 | 10 |
| 1 | 1:0,3 | 3 | soluble | Pas de bande isocyanate | 1 410 | - | 11,8 | - | 6,6 |
| 2 | 1:0,5 | 3 | soluble | Pas de bande isocyanate | 2550 | - | 105 | - | 60 |
| 3 | 1:0,75 | 12 | soluble | Pas de bande isocyanate | 4060 | 620 | 563 | 163 | 148 |

## Revendications

1. Composé de formule (I-4) suivante : 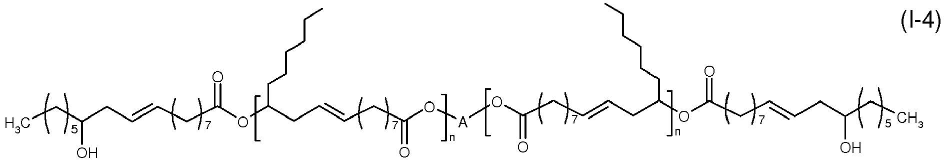 dans laquelle :
- n est compris de 1 à 20 ; et
- A représente un radical alkylène divalent linéaire ou ramifié, comprenant de 1 à 10 atomes de carbone, et étant interrompu par un ou plusieurs atomes d'oxygène.

2. Composé selon la revendication 1 dans lequel A représente un radical alkylène comprenant 3 ou 4 atomes de carbone.

3. Composé selon l'une quelconque des revendications 1 ou 2, dans lequel A représente un radical (poly)propylèneglycol.

4. Polymères tels qu'obtenus par polymérisation d'un composé tel que défini dans l'une quelconque des revendications 1 à 3, et d'un (poly)isocyanate.

## Patentansprüche

1. Verbindung der folgenden Formel (I-4): 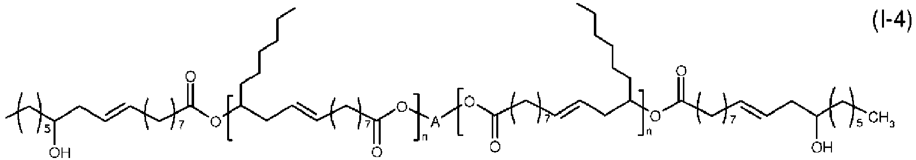 wobei:
- n zwischen einschließlich 1 und 20 liegt; und
- A ein lineares oder verzweigtes zweiwertiges Alkylenradikal darstellt, das 1 bis 10 Kohlenstoffatome aufweist und mittels eines oder mehrerer Sauerstoffatome unterbrochen ist.

2. Verbindung gemäß Anspruch 1, wobei A ein Alkylenradikal, das 3 oder 4 Kohlenstoffatome aufweist, darstellt.

3. Verbindung gemäß Anspruch 1 oder 2, wobei A ein (Poly)propylenglycol-Radikal darstellt.

4. Polymere, wie sie durch Polymerisation einer Verbindung wie gemäß einem der Ansprüche 1 bis 3 definiert und eines (Poly)isocyanats gewonnen werden.

## Claims

1. The compound of the following formula (I-4): 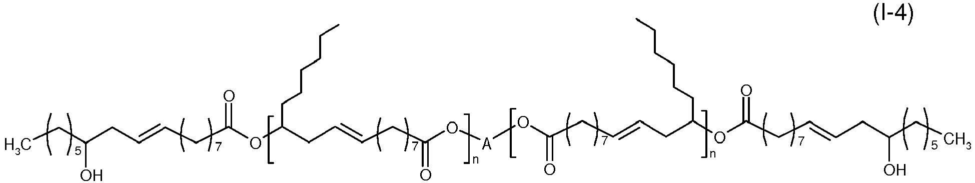 wherein:
- n is comprised from 1 to 20; and
- A represents a linear or branched divalent alkylene radical, comprising from 1 to 10 carbon atoms, and optionally interrupted by one or more hetero-atoms, and more particularly by one or more oxygen atoms..

2. The compound according to claim 1, wherein A represents an alkylene radical comprising 3 or 4 carbon atoms.

3. The compound according to any of claims 1 or 2, wherein A represents a (poly)propyleneglycol radical.

4. Polymers as obtained by polymerization of a compound as defined in any of claims 1 to 3 and of a (poly)isocyanate.
